# EUROPEAN PATENT SPECIFICATION

(11) **EP 2 814 967 B1**
(45) Date of publication and mention of the grant of the patent: **03.06.2026**
(21) Application number: 13709582.4
(22) Date of filing: 18.02.2013
(51) Int. Cl.: C12N 15/82, A01H 5/00

(54) **IMPROVING DROUGHT RESISTANCE IN PLANTS: UPL3**
VERBESSERUNG DER DÜRRERESISTENZ BEI PFLANZEN: UPL3
AMÉLIORATION DE LA RÉSISTANCE DE PLANTES À LA SÉCHERESSE : UPL3

(30) Priority: 17.02.2012 US 201261599961 P
(43) Date of publication of application: 24.12.2014
(73) Proprietor: Keygene N.V., 6700 AE Wageningen (NL)
(72) Inventor: DESLATTES MAYS, Anne, NL-6700 AE Wageningen (NL); VAN HULTEN, Marieke Helena Adriana, NL-6700 AE Wageningen (NL); DIXIT, Shital Anilkumar, NL-6700 AE Wageningen (NL); BLOM, Evert-Jan, NL-6700 AE Wageningen (NL); MUNKVOLD, Jesse David, Rockville, Maryland 20850 (US); DILEO, Matthew Vitabile, Silver Spring, Maryland 20901 (US); DE VOS, Martin, NL-6700 AE Wageningen (NL)
(74) Representative: Nederlandsch Octrooibureau
(86) International application number: PCT/NL2013/050101
(87) International publication number: WO 2013/122472

(56) References cited:
- US-A1- 2004 031 072
- US-A1- 2004 123 343
- DOWNES BRIAN P ET AL: "The HECT ubiquitin-protein ligase (UPL) family in Arabidopsis: UPL3 has a specific role in trichome development", THE PLANT JOURNAL, BLACKWELL SCIENTIFIC PUBLICATIONS, OXFORD, GB, vol. 35, no. 6, 1 September 2003 (2003-09-01), pages 729 - 742, XP002433599, ISSN: 0960-7412, DOI: 10.1046/J.1365-313X.2003.01844.X
- FENG QIN ET AL: "Arabidopsis DREB2A-interacting proteins function as RING E3 ligases and negatively regulate plant drought stress-responsive gene expression", THE PLANT CELL, AMERICAN SOCIETY OF PLANT BIOLOGISTS, US, vol. 20, no. 6, 1 January 2008 (2008-01-01), pages 1693 - 1707, XP002656955, ISSN: 1040-4651, DOI: 10.1105/TPC.107.057380
- SEOK KEUN CHO ET AL: "Arabidopsis PUB22 and PUB23 are homologous U-box E3 ubiquitin ligases that play combinatory roles in response to drought stress", THE PLANT CELL, AMERICAN SOCIETY OF PLANT BIOLOGISTS, US, vol. 20, no. 7, 1 July 2008 (2008-07-01), pages 1899 - 1914, XP002656954, ISSN: 1040-4651, DOI: 10.1105/TPC.108.060699

## Description

### Technical Field

The present invention relates to a new method for increasing drought resistance of a plant. The method encompasses the impairment of the expression of a gene or genes or functional protein(s) in said plant. In comparison to a plant not manipulated to impair the expression of said gene(s) or functional protein(s), the plants display improved drought resistance. Also described are plants and plant product that can be obtained by the method according to the invention.

### Background of the invention

Abiotic stresses, such as drought, salinity, extreme temperatures, chemical toxicity and oxidative stress are threats to agriculture and it is the primary cause of crop loss worldwide (Wang et al. (2003) Planta 218(1) 1 -14).

In the art, several reports are available dealing with the biochemical, molecular and genetic background of abiotic stress (Wang et al. (2003) Planta 218(1) 1 -14 or Kilian et al (2007) Plant J 50(2) 347-363). Plant modification to deal with abiotic stress is often based on manipulation of genes that protect and maintain the function and structure of cellular components. However, due to the genetically complex responses to abiotic stress conditions, such plants appear to be more difficult to control and engineer. Wang, (Wang et al. (2003) Planta 218(1) 1 -14), inter alia, mentions that one of the strategies of engineering relies on the use of one or several genes that are either involved in signalling and regulatory pathways, or that encode enzymes present in pathways leading to the synthesis of functional and structural protectants, such as osmolytes and antioxidants, or that encode stress-tolerance-conferring proteins.

Although improvements in providing abiotic stress tolerant plants have been reported, the nature of the genetically complex mechanisms underlying it provides a constant need for further improvement in this field. For example, it has been reported that genetically transformed drought tolerant plants generally may exhibit slower growth and reduced biomass (Serrano et al (1999) J Exp Bot 50:1023-1036) due to an imbalance in development and physiology, thus having significant fitness cost in comparison with plants that are not transformed (Kasuga et al. (1999) Nature Biot. Vol. 17; Danby and Gehring (2005) Trends in Biot. Vol.23 No.11).

Several biotechnological approaches are proposed in order to obtain plants growing under stress conditions. Plants with increased resistance to salt stress are for example disclosed in WO03/020015. This document discloses transgenic plants that are resistant to salt stress by utilizing 9-cis-epoxycarotenoid dioxygenase nucleic acids and polypeptides.

Plants with increased drought tolerance are disclosed in, for example, US 2009/0144850, US 2007/0266453, and WO 2002/083911. US2009/0144850 describes a plant displaying a drought tolerance phenotype due to altered expression of a DR02 nucleic acid. US 2007/0266453 describes a plant displaying a drought tolerance phenotype due to altered expression of a DR03 nucleic acid and WO 2002/083911 describes a plant having an increased tolerance to drought stress due to a reduced activity of an ABC transporter which is expressed in guard cells. Another example is the work by Kasuga and co-authors (1999), who describe that overexpression of cDNA encoding DREB1A in transgenic plants activated the expression of many stress tolerance genes under normal growing conditions and resulted in improved tolerance to drought, salt loading, and freezing. However, the expression of DREB1A also resulted in severe growth retardation under normal growing conditions (Kasuga (1999) Nat Biotechnol 17(3) 287-291). There remains a need for new, alternative and/or additional methodology for increasing resistance to abiotic stress, in particular abiotic stress like drought.

It is an object of the current invention to provide for new methods to increase drought resistance in a plant. With such plant it is, for example, possible to produce more biomass and/or more crop and plant product derived thereof if grown under conditions of low water availability/drought in comparison with plants not subjected to the method according to the invention.

### Summary

The invention is defined by the claims. Provided herein is a method for producing a plant having improved drought resistance compared to a control plant, comprising the step of impairing expression of a UPL protein in a plant, said UPL protein comprising an amino acid sequence comprising at least one Pfam HECT domain according to PF00632 and at least one Superfamily ARM repeat according to model SSF48371, and optionally regenerating said plant.

In an aspect, the present invention provides a method for producing a plant having improved drought resistance compared to a control plant, comprising the step of impairing expression of functional UPL3 protein in a plant, plant cell or plant protoplast, wherein said functional UPL3 protein is encoded by a nucleic acid comprising a nucleic acid sequence having at least 80% identity with SEQ ID NO:1, or wherein the UPL3 protein has the amino acid sequence of at least one of SEQ iD NO: 2, 6 and 8, wherein the plant, plant protoplast or plant cell belongs to at least one of the *Brassicaceae* family and the *Solanaceae* family, and wherein the UPL3 expression is impaired by at least one of: i) a step of mutating a nucleic acid sequence encoding said endogenous UPL3 protein; and ii) a step of gene silencing, and wherein the method further comprises the steps of regenerating said plant; and determining improved drought resistance of said plant as compared to the control plant.

Said functional UPL3 protein may comprise an amino acid sequence comprising at least one Pfam HECT domain according to PF00632 and at least one Superfamily ARM repeat according to model SSF48371.

The functional UPL3 protein may be a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL4 protein is not expressed.

Further described herein is a method for producing a plant having improved drought resistance compared to a control plant, comprising the step of impairing expression of functional UPL3 protein in a plant, plant cell or plant protoplast, wherein said functional UPL3 protein comprises an amino acid sequence having at least one Pfam HECT domain according to PF00632 and at least one Superfamily ARM repeat according to model SSF48371, and optionally regenerating said plant.

Further described herein is a method for producing a plant having improved drought resistance compared to a control plant, comprising the step of impairing expression of functional UPL3 protein, wherein said functional UPL3 protein is encoded by a nucleic acid sequence comprising a nucleic acid sequence having at least 60% identity with the nucleic acid sequence of SEQ ID NO:1, and optionally regenerating said plant.

The functional UPL3 protein may be a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed.

The step of impairing expression of functional UPL3 protein may comprise mutating a nucleic acid sequence encoding said functional UPL3 protein. Mutating said nucleic acid sequence may involve an insertion, a deletion and/or substitution of at least one nucleotide. The step of impairing expression may comprise gene silencing. The step of impairing expression may comprise impairing expression of two or more functional UPL3 proteins in said plant.

The method may further comprise the step of producing a plant or plant product from the plant having improved drought resistance.

Further described herein is the use of an amino acid sequence having at least 30% identity with the amino acid sequence of SEQ ID NO:2 or a nucleic acid sequence having at least 60% identity with the nucleic acid sequence of SEQ ID NO: 1 in the screening for drought resistance in plants.

Further described herein is the use of an UPL3 amino acid sequence having SEQ ID NO:2 or a UPL3 nucleic acid sequence of SEQ ID NO:1 in the screening for drought resistance in *Arabidopsis thaliana* plants.

Further described herein is the use of at least part of a UPL3 nucleic acid sequence of SEQ ID NO:1 or at least part of an UPL3 amino acid sequence of SEQ ID NO:2 as a marker for breeding drought resistant *Arabidopsis thaliana* plants.

Further described herein is the use of a functional UPL3 protein as defined herein for modulating, preferably increasing, drought resistance of a plant.

Further described herein is a plant, plant cell, or plant product wherein expression of functional UPL3 protein is impaired, wherein the functional UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed for growing under drought stress conditions, wherein said drought stress conditions cause a control plant, plant cell or plant product wherein expression of said functional UPL3 protein is not impaired to show signs of drought stress such as wilting signs earlier than the plant, plant cell, or plant product wherein expression of functional UPL3 protein is impaired.

Further described herein is a *Solanum lycopersicum, Gossypium hirsutum, Glycine max, Triticum* spp., *Hordeum vulgare., Avena sativa, Sorghum bicolor, Secale cereale,* or *Brassica napus* plant, plant cell, or plant product wherein expression of functional UPL3 protein is impaired, wherein the functional UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed. Said plant, plant cell, or plant product may comprise a disrupted endogenous UPL3 gene.

### Brief Description of the Drawings

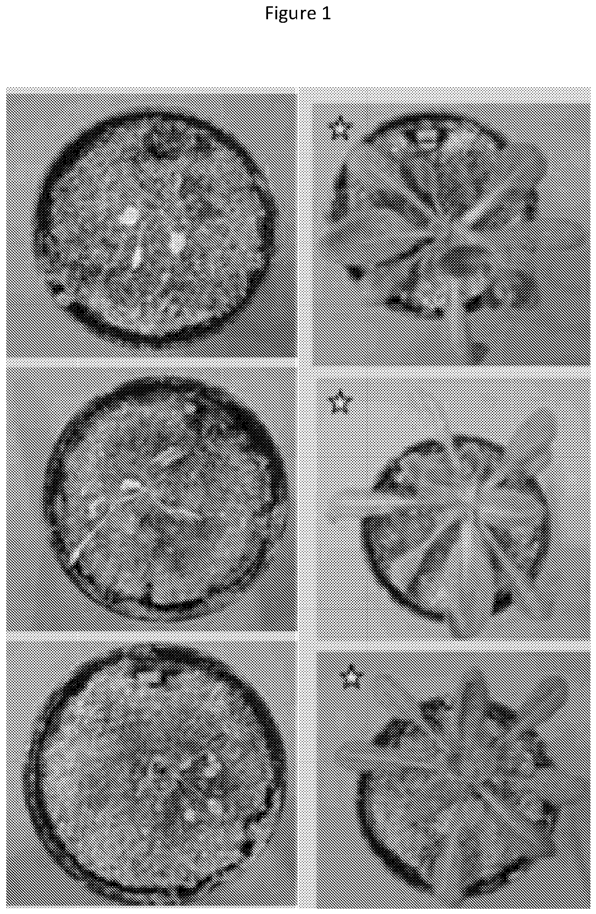

Figure 1 shows the results of a typical experiment described in the Examples 1 and 2.

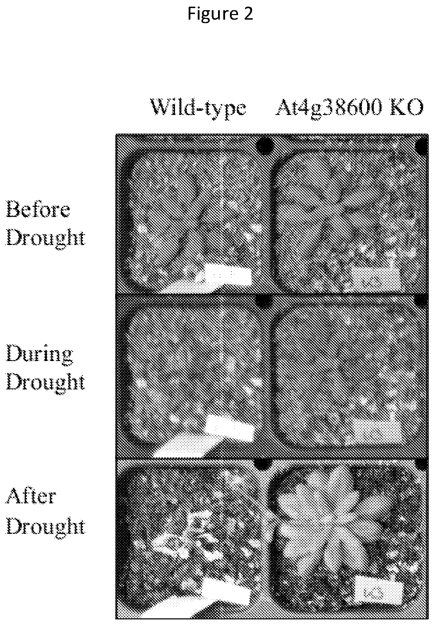

Figure 2 shows the drought resistant phenotype of the UPL3 knockout *(Arabidopsis* At4g38600 insertion mutant) as compared to the drought sensitive phenotype of a control (wild-type) plant.

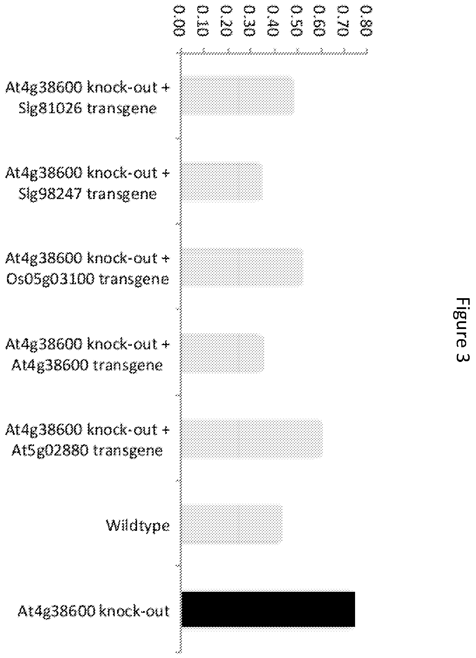

Figure 3 shows drought survival of At4g38600-insertion mutant (UPL3). The *Arabidopsis thaliana* At4g38600 insertion mutant survived drought significantly better (p < 0.05) than wild-type (Col-0) plants or At4g38600 insertion mutants complemented with the coding sequence (CDS) of At4g38600 (SEQ ID NO: 1; positive control) and homologs from *Arabidopsis thaliana* (SEQ ID NO:3), *Solanum lycopersicum* (SEQ ID NO:5 and 7) or *Oryza sativa* (SEQ ID NO:9). This figure demonstrates that an insertion mutation in the UPL3 gene produces a drought resistant phenotype. Moreover, it also indicates that homologs of this gene from monocot and dicot species operate to restore the normal drought-susceptible phenotype. Hence, these homologs perform the same function in drought tolerance in their respective crop species. The observation that both monocot and dicot UPL3 genes can restore drought susceptibility when inserted into the UPL3 insertion mutant of *Arabidopsis* suggests that a reduced activity of the protein encoded by the UPL3 gene renders drought tolerant phenotypes throughout the entire plant kingdom. Hence, prediction of UPL3 (based on homology searches and characteristic domain [HECT] and Armadillo repeat sequences) will allow identification of plant UPL3 homologs in plant species. Subsequently, one can use well-known methods to reduce protein activity of these plant homologs (e.g. mutagenesis, TDNA or transposon insertion, RNAi, etc) to obtain drought resistant plants. Grey bars have significantly lower values (p < 0.05) than black bars.

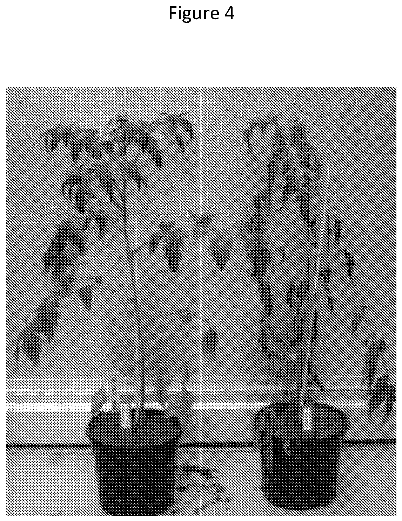

Figure 4 shows the drought phenotype of a tomato *(Solanum lycopersicum)* UPL3-mutant. A segregating M2 population containing homozygous, heterozygous and wild-type allele were used for a drought experiment. The photograph - taken 21 days after initiation of the drought treatment - shows a wild-type tomato plant (right) and a plant carrying the V158E mutation in SIg98247 (left). Drought tolerant phenotype and survival of the drought treatment was significantly better (p < 0.1) for the plant carrying the V158E mutation in SIg98247 compared to the wild-type allele, indicating that this alteration of the protein leads to a drought tolerant phenotype in tomato.

### Definitions

In the following description and examples, a number of terms are used. In order to provide a clear and consistent understanding of the specification and claims, including the scope to be given to such terms, the following definitions are provided. Unless otherwise defined herein, all technical and scientific terms used have the same meaning as commonly understood by one of ordinary skill in the art to which this invention belongs.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al.., Molecular Cloning. A Laboratory Manual, 2nd Edition, Cold Spring Harbor Laboratory Press, Cold Spring Harbor, N. Y., 1989; Ausubel et al., Current Protocols in Molecular Biology, John Wiley & Sons, New York, 1987 and periodic updates; and the series Methods in Enzymology, Academic Press, San Diego.

In this document and in its claims, the verb "to comprise" and its conjugations is used in its non-limiting sense to mean that items following the word are included, but items not specifically mentioned are not excluded. It encompasses the verbs "consisting essentially of" as well as "consisting of".

As used herein, the singular forms "a," "an" and "the" include plural referents unless the context clearly dictates otherwise. For example, a method for isolating "a" DNA molecule, as used above, includes isolating a plurality of molecules (e.g. 10's, 100's, 1000's, 10's of thousands, 100's of thousands, millions, or more molecules).

Aligning and alignment: With the term "aligning" and "alignment" is meant the comparison of two or more nucleotide sequences based on the presence of short or long stretches of identical or similar nucleotides. Several methods for alignment of nucleotide sequences are known in the art, as will be further explained below.

"Expression of a gene" refers to the process wherein a DNA region, which is operably linked to appropriate regulatory regions, particularly a promoter, is transcribed into an RNA, which is biologically active, i.e. which is capable of being translated into a biologically active protein or peptide (or active peptide fragment). "Ectopic expression" refers to expression in a tissue in which the gene is normally not expressed. "Expression of a protein" is used herein interchangeably with the term expression of a gene. It refers to the process in which a DNA region, which is operably linked to appropriate regulatory regions, particularly a promoter, is transcribed into an mRNA and which is subsequently translated into a protein or peptide (or active peptide fragment).

"Functional", in relation to UPL3 proteins (or variants, such as orthologs or mutants, and fragments), refers to the capability of the gene and/or encoded protein to modify the (quantitative and/or qualitative) drought resistance, e.g., by modifying the expression level of the gene (e.g. by overexpression or silencing) in a plant. For example, the functionality of a UPL3 protein obtained from plant species X can be tested by various methods. Preferably, if the protein is functional, silencing of the gene encoding the protein in plant species X, using e.g. gene silencing vectors, will lead to a improved drought resistance as can be tested as explained herein in detail. Also, complementation of a UPL3 knockout with a functional UPL3 protein (or UPL4 gene) will be capable of restoring or conferring the characteristic, in this case will restore drought sensitivity. The skilled person will have no difficulties in testing functionality.

The term "gene" means a DNA sequence comprising a region (transcribed region), which is transcribed into an RNA molecule (e.g. an mRNA) in a cell, operably linked to suitable regulatory regions (e.g. a promoter). A gene may thus comprise several operably linked sequences, such as a promoter, a 5' leader sequence comprising e.g. sequences involved in translation initiation, a (protein) coding region (cDNA or genomic DNA) and a 3' non-translated sequence comprising e.g. transcription termination sequence sites.

The term "cDNA" means complementary DNA. Complementary DNA is made by reverse transcribing RNA into a complementary DNA sequence. cDNA sequences thus correspond to RNA sequences that are expressed from genes. As mRNA sequences when expressed from the genome can undergo splicing, i.e. introns are spliced out of the mRNA and exons are joined together, before being translated in the cytoplasm into proteins, it is understood that expression of a cDNA means expression of the mRNA that encodes for the cDNA. The cDNA sequence thus may not be identical to the genomic DNA sequence to which it corresponds as cDNA may encode only the complete open reading frame, consisting of the joined exons, for a protein, whereas the genomic DNA encodes and exons interspersed by intron sequences . Genetically modifying a gene which encodes the cDNA may thus not only relate to modifying the sequences corresponding to the cDNA, but may also involve mutating intronic sequences of the genomic DNA and/or other gene regulatory sequences of that gene, as long as it results in the impairment of gene expression.

"Identity" is a measure of the identity of nucleotide sequences or amino acid sequences. In general, the sequences are aligned so that the highest order match is obtained. "Identity" *per se has* an art-recognized meaning and can be calculated using published techniques. See, e.g.: (COMPUTATIONAL MOLECULAR BIOLOGY, Lesk, A. M., ed., Oxford University Press, New York, 1988; BIOCOMPUTING: INFORMATICS AND GENOME PROJECTS, Smith, D. W., ed., Academic Press, New York, 1993; COMPUTER ANALYSIS OF SEQUENCE DATA, PART I, Griffin, A. M., and Griffin, H. G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER; Gribskov, M. and Devereux, J., eds., M Stockton Press, New York, 1991). While a number of methods exist to measure identity between two polynucleotide or polypeptide sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in GUIDE TO HUGE COMPUTERS, Martin J. Bishop, ed., Academic Press, San Diego, 1994, and Carillo, H., and Lipton, D., SIAM J. Applied Math (1988) 48:1073. Methods to determine identity and similarity are codified in computer programs. Preferred computer program methods to determine identity and similarity between two sequences include, but are not limited to, GCS program package (Devereux, J., et al., Nucleic Acids Research (1984) 12(1):387), BLASTP, BLASTN, FASTA (Atschul, S. F. et al., J. Molec. Biol. (1990) 215:403). The percentage identity is preferably determined over the entire length of the nucleotide or amino acid sequence.

As an illustration, by a polynucleotide having a nucleotide sequence having at least, for example, 95% "identity" to a reference nucleotide sequence encoding a polypeptide of a certain sequence it is intended that the nucleotide sequence of the polynucleotide is identical to the reference sequence except that the polynucleotide sequence may include up to five point mutations per each 100 nucleotides of the reference polypeptide sequence. Hence, the percentage of identity of a nucleotide sequence to a reference nucleotide sequence is to be calculated over the entire length of the reference nucleotide sequence. In other words, to obtain a polynucleotide having a nucleotide sequence at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. These mutations of the reference sequence may occur at the 5' or 3' terminal positions of the reference nucleotide sequence, or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO: 2 is intended that the amino acid sequence of the polypeptide is identical to the reference sequence except that the polypeptide sequence may include up to five amino acid alterations per each 100 amino acids of the reference amino acid of SEQ ID NO: 2. Hence, the percentage of identity of an amino acid sequence to a reference amino acid sequence is to be calculated over the entire length of the reference amino acid sequence. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

A nucleic acid according to the method of the claimed invention may include any polymer or oligomer of pyrimidine and purine bases, preferably cytosine, thymine, and uracil, and adenine and guanine, respectively (*See* Albert L. Lehninger, Principles of Biochemistry, at 793-800 (Worth Pub. 1982) The claimed invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogenous or homogenous in composition, and may be isolated from naturally occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked may mean that the DNA sequences being linked are contiguous.

"Plant" refers to either the whole plant or to parts of a plant, such as cells, tissue or organs (e.g. pollen, seeds, gametes, roots, leaves, flowers, flower buds, anthers, fruit, etc.) obtainable from the plant, as well as derivatives of any of these and progeny derived from such a plant by selfing or crossing. "Plant cell(s)" include protoplasts, gametes, suspension cultures, microspores, pollen grains, etc., either in isolation or within a tissue, organ or organism.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. Optionally the term "promoter" includes herein also the 5' UTR region (5' Untranslated Region) (e.g. the promoter may herein include one or more parts upstream (5') of the translation initiation codon of a gene, as this region may have a role in regulating transcription and/or translation. A "constitutive" promoter is a promoter that is active in most tissues under most physiological and developmental conditions. An "inducible" promoter is a promoter that is physiologically (e.g. by external application of certain compounds) or developmentally regulated. A "tissue specific" promoter is only active in specific types of tissues or cells. A "promoter active in plants or plant cells" refers to the general capability of the promoter to drive transcription within a plant or plant cell. It does not make any implications about the spatio-temporal activity of the promoter.

The terms "protein" or "polypeptide" are used interchangeably and refer to molecules consisting of a chain of amino acids, without reference to a specific mode of action, size, 3 dimensional structure or origin. A "fragment" or "portion" of a protein may thus still be referred to as a "protein". An "isolated protein" is used to refer to a protein which is no longer in its natural environment, for example *in vitro* or in a recombinant bacterial or plant host cell.

"Transgenic plant" or "transformed plant" refers herein to a plant or plant cell having been transformed, e.g. by the introduction of a non-silent mutation in an endogenous gene or part there of. Such a plant has been genetically modified to introduce for example one or more mutations, insertions and/or deletions in the gene and/or insertions of a gene silencing construct in the genome. A transgenic plant cell may refer to a plant cell in isolation or in tissue culture, or to a plant cell contained in a plant or in a differentiated organ or tissue, and both possibilities are specifically included herein. Hence, a reference to a plant cell in the description or claims is not meant to refer only to isolated cells or protoplasts in culture, but refers to any plant cell, wherever it may be located or in whatever type of plant tissue or organ it may be present.

Targeted nucleotide exchange (TNE) is a process by which a synthetic oligonucleotide, partially complementary to a site in a chromosomal or an episomal gene directs the reversal of a single nucleotide at a specific site. TNE has been described using a wide variety of oligonucleotides and targets. Some of the reported oligonucleotides are RNA/DNA chimeras, contain terminal modifications to impart nuclease resistance.

As used herein, the term "drought stress" or "drought" refers to a sub-optimal environmental condition associated with limited availability of water to a plant. Limited availability of water may occur when for instance rain is absent or lower and/or when the plants are watered less frequently than required. Limited water availability to a plant may also occur when for instance water is present in soil, but can not efficiently be extracted by the plant. For instance, when soils strongly bind water or when the water has a high salt content, it maybe more difficult for a plant to extract the water from the soil. Hence, many factors can contribute to result in limited availability of water, i.e. drought, to a plant. The effect of subjecting plants to "drought" or "drought stress" may be that plants do not have optimal growth and/or development. Plants subjected to drought may have wilting signs. For example, plants may be subjected to a period of at least 15 days under specific controlled conditions wherein no water is provided, e.g. without rain fall and/or watering of the plants.

The term "improved drought resistance" refers to plants which, when provided with improved drought resistance, when subjected to drought or drought stress do not show effects or show alleviated effects as observed in plants not provided with improved drought resistance. A normal plant has some level of drought resistance. It can easily be determined whether a plant has improved drought resistant by comparing a control plant with a plant provided with improved drought resistance under controlled conditions chosen such that in the control plants signs of drought can be observed after a certain period, i.e. when the plants are subjected to drought or drought stress. The plants with improved drought resistance will show less and/or reduced signs of having been subjected to drought, such as wilting, as compared to the control plants. The skilled person knows how to select suitable conditions such as for example the controlled conditions in the examples. When a plant has "improved drought resistance", it is capable of sustaining normal growth and/or normal development when being subjected to drought or drought stress would otherwise would have resulted in reduced growth and/or reduced development of normal plants. Hence, "improved drought resistance" is a relative term determined by comparing plants, whereby the plant most capable of sustaining (normal) growth under drought stress is a plant with "improved drought resistant" plant. The skilled person is well aware how to select appropriate conditions to determine drought resistance of a plant and how to measure signs of droughts, such as described in for example manuals provided by the IRRI, Breeding rice for drought prone environments, Fischer et al., 2003, and by the CIMMYT, Breeding for drought and nitrogen stress tolerance in maize: from theory to practice, Banzinger et al, 2000. Examples of methods determining improved drought resistance in plants are provided in Snow and Tingey, 1985, Plant Physiol, 77, 602-7 and Harb et al., Analysis of drought stress in Arabidopsis, AOP 2010, Plant Physiology Review, and as described in the example section below.

### Detailed description

The current invention relates to the improvement of drought resistance of a plant by impairing the expression of a functional UPL3 protein in said plant. The improvement is relative to a control plant, in which such modification has not been introduced or is not present and in which expression of a functional UPL3 protein is not impaired. In other words, modified plant is, in comparison to the control plant, i.e. non-modified plant, better able to grow and survive under conditions of reduced water availability, (temporary) water-deprivation or conditions of drought. It is understood that according to the invention modifying, e.g., impairing, expression of functional UPL3 protein may involve genetic modification, e.g., of UPL3 gene expression, or targeted nucleotide exchange.

Genetic modification includes introducing mutations, insertions, deletions in the nucleic acid sequence of interest and/or insertion of gene silencing constructs into a genome of a plant or plant cell that target the nucleic acid sequence of interest. Genetically modifying a nucleic acid sequence, e.g., a gene, which encodes the mRNA may not only relate to modifying exon sequences corresponding to the mRNA sequence, but may also involve mutating intronic sequences of genomic DNA and/or (other) gene regulatory sequences of that nucleic acid sequence, e.g., gene.

In the context of the present invention, the functional UPL3 protein may be a protein that, when expressed in an *Arabidopsis thaliana* T-DNA insertion line having a disrupted endogenous UPL4 gene, such as an At4g38600 knockout line, e.g., SALK_037636C (http://www.arabidopsis.org/servlets/TairObject?type=stock&id=3501631890) recited herein, results in a plant with an impaired drought resistance compared to the drought resistance of said *Arabidopsis thaliana* T-DNA insertion line having a disrupted endogenous UPL3 gene, e.g., an At4g38600 knockout line, e.g., SALK_037636C, in which said functional UPL3 protein is not expressed.

The term "disrupted endogenous UPL3 gene" as used herein refers to a UPL3 gene naturally present in the genome of a plant which is disrupted, e.g., interrupted, e.g., by means of a T-DNA insertion into said UPL3 gene. Disruption of said endogenous UPL3 gene may result in the absence of expression of said endogenous UPL3 gene, and thus in the absence of endogenous UPL3 protein (either functional or non-functional).

The term "control plant" as used herein refers to a plant of the same species, preferably of the same variety, preferably of the same genetic background.

The current invention also relates to the modulation of drought resistance of a plant by modifying the expression of functional UPL3 protein in said plant. The modulation is relative to a similar plant (preferably of the same species and/or variety, and preferably of the same genetic background) in which such modification has not been introduced or is not present.

Provided herein is a method for producing a plant having improved drought resistance compared to a control plant, comprising the step of impairing expression of a UPL protein in a plant, said UPL protein comprising an amino acid sequence comprising at least one Pfam HECT domain according to PF00632 and at least one Superfamily ARM repeat according to model SSF48371.

In another aspect, described herein is a method for producing a plant having improved drought resistance compared to a control plant, the method comprising the step of impairing the expression of functional UPL3 protein in said plant.

"Impairing expression of a functional UPL3 protein" as used herein may mean that the expression of the UPL3 gene has been impaired, and/or that expression of the UPL3 gene is normal but translation of the resulting mRNA is inhibited or prevented (for example, by RNA interference), and/or that the amino acid sequence of UPL3 protein has been altered such that its ubiquitin protein ligase specific activity is reduced compared to the ubiquitin protein ligase specific activity of the protein as depicted in SEQ ID NO:2, preferably under physiological conditions, particularly identical physiological conditions. Alternatively, a UPL3 protein may become non-functional by simultaneous expression of an antibody specifically binding to said UPL3 protein, thereby reducing its specific activity. The ubiquitin protein ligase specific activity of a UPL3 protein may be considered "reduced" if the ubiquitin protein ligase specific activity of such protein is statistically significantly less than the ubiquitin protein ligase specific activity of the protein as depicted in SEQ ID NO:2. The ubiquitin protein ligase specific activity of a UPL3 protein may, for example, be reduced by at least 5%, 10%, 15%, 20%, 25%, 30%, 35%, 40%, 45%, 50%, or more. Reduced expression of the endogenous UPL3 gene of a plant may be accomplished by altering the promoter sequence, for example, using targeted mutagenesis.

It is believed by the current inventors that impairing expression (e.g. by reducing, repressing or deleting expression and/or activity) of functional UPL3 protein leads to the absence or a reduced level of functional UPL3 protein, either as a consequence of low expression, e.g. via RNA interference, or as a consequence of decreased activity/functionality of the UPL3 protein, or one or more of the above, and that said absence or reduced level of functional UPL3 protein leads to decreased need for water or improved resistance to drought of said plant.

Ubiquitin Protein Ligase proteins (UPLs) are known to be involved in the selective degradation of regulatory proteins in both yeast and animals (Huibregtse et al. (1995) Proc. Natl. Acad. Sci. USA 92, 2563-2567; Pickart (2001) Annu. Rev. Biochem. 70, 503-533). Proteins committed for degradation are modified with a chain of multiple Ubiquitins and are then recognized by the 26S proteasome. An important class of these Ubiquitin Protein Ligase proteins is formed by the HECT E3s, which comprise a conserved 350-amino acid domain called the HECT domain at the C-terminal end (based on its homology to the C-terminus of human E6-Associated Protein (E6-AP) (Huibregtse et al. (1995) Proc. Natl. Acad. Sci. USA, 92, 2563-2567). The HECT domain includes a highly conserved region surrounding the positionally invariant cystein required to catalyze Ubiquitin transfer.

According to Downes et al. (2003, Plant J 35, 729-742), plants also contain HECT E3s, with seven present in Arabidopsis: UPL1, UPL2, UPL3, UPL4, UPL5, UPL6, and UPL7. Downes et al. further describe that UPL1, UPL2, UPL3, UPL4, UPL5, UPL6, and UPL7 can be grouped by structure into four subfamilies based on intron/exon positions of the corresponding genes, protein sequence and length, and the presence of additional protein motifs upstream of the HECT domain: UPL1/2, UPL3/4, UPL5, and UPL6/7. The presence of a variety of domains upstream of the HECT domain suggests that individual members of the UPL1-UPL7 family have distinct sets of targets and functions (see Downes et al. 2003 The Plant Journal, 35, 729-742, in particular Figure 1 thereof, for more information on the distinct characteristics of the different UPL proteins).

In *Arabidopsis thaliana,* Ubiquitin Protein Ligase 4 can be distinguished from Ubiquitin Protein Ligase 3 for instance by the absence of a 225-residue region 650 amino acids from the C-terminus of Ubiquitin Ligase 4 (Downes et al. (2003) Plant J 35, 729-742)

Ubiquitin Protein Ligase 4 as found in *Arabidopsis thaliana* has been reported by other to have approximately 54% amino acid sequence identity to Ubiquitin Protein Ligase 3 (Downes et al. (2003) Plant J 35, 729-742). The locus name of the Ubiquitin Protein Ligase 3 is At4g38600/At4g38610, and the ORF name is F20M13.160/F20M13.170 (both according to http://www.uniprot.org/uniprot/Q6WWW4).

The UPL3 protein of *Arabidopsis thaliana* comprises 1888 amino acids (as depicted in SEQ ID NO:2). The cDNA encoding the UPL3 protein of Arabidopsis thaliana comprises 4506 nucleotides (depicted in SEQ ID NO:1).

A "UPL3 protein" as used herein comprises the protein depicted in SEQ ID NO:2, as well as fragments and variants thereof. Variants of a UPL3 protein include, for example, proteins having at least 30%, 35%, 40%, 50%, 60%, 70%, 75%, 80%, 85%, 90%, 95%, 96%, 97%, 98%, 99%, or more, such as 100%, amino acid sequence identity, preferably over the entire length, to SEQ ID NO:2. Amino acid sequence identity is determined by pairwise alignment using the Needleman and Wunsch algorithm and GAP default parameters as defined above. A UPL3 protein may be considered functional if it has ubiquitin protein ligase activity.

An *Arabidopsis thaliana* plant having a T-DNA insertion in the gene encoding UPL3 is known from Downes et al. ((2003) Plant J. 35, 729-742). This UPL3 mutant shows aberrant trichome development.

In another aspect there is provided for a method for producing a plant having improved drought resistance, the method comprising the step of impairing the expression in said plant of a gene encoding a UPL3 protein.

"Impaired expression "according to the present invention denotes the absence or reduced presence of a functional UPL3 protein and variants thereof comprising an amino acid sequence with more than 40%, 50%, 60%, 70%, 80%, 90%, 95 % sequence identity therewith. It also denotes the absence of lowered presence of proteins described herein that comprise at least one Pfam HECT domain PF00632 and at least one Superfamily ARM repeat model SSF48371. A skilled person is well aware of the many mechanism available to him in the art to impair the expression of a gene or protein at, for example, the transcriptional level or the translational level.

In another aspect there is provided for a method for increasing drought resistance of a plant, the method comprising the step of impairing the expression in said plant of a gene or a protein, wherein the amino acid sequence (or protein) encoded by said gene comprises at least one Pfam HECT domain (PF00632) and at least one Superfamily ARM repeat (model SSF48371), as determined as described below. It is understood that the phrase "at least one Superfamily ARM repeat model SSF48371" comprises the four Armadillo repeat sequences from the UPL3 gene as depicted in SEQ ID NO:1. Thus, the phrase "at least one Superfamily ARM repeat model SSF48371" means to comprise the four Armadillo repeat sequences.

As used herein "Pfam" or "PFAM" refers to a large collection of multiple sequence alignments and hidden Markov models covering many common protein families, and is available from http://pfam.sanger.ac.uk/. The Pfam database contains a large collection of protein families, each represented by multiple alignments. These alignments have been used to build hidden Markov models (HMMs) for each protein domain family. The alignments represent evolutionary conserved structures and the presence of a domain in a protein of interest can be indicative towards its biological function. Profile hidden Markov models (profile HMMs) built from the Pfam alignments are useful for automatically recognizing that a new protein belongs to an existing protein family even if the homology by alignment appears to be low. Other proteins in the same protein family are identified by querying the amino acid sequence of a protein sequence against the Hidden Markov Model using HMMER software. The HMMER software (version 3.0 from http://hmmer.janelia.org/) is able to use this HMM to search for a presence of this domain in new sequences. Potential candidate proteins hits were derived by taking into account only HMMER hits in their sequences that were above the default inclusion threshold.

Pfam version 24.0 (October 2009) contains alignments and models for 11912 protein families (see The Pfam protein families database: R.D. Finn, et al Nucleic Acids Research (2010) Database Issue 38:D211-222). Pfam is based on a sequence database called Pfamseq, which is based on UniProt release 15.6 (Swiss-Prot release 57.6 and SP-TrEMBL release 40.6).

The alignments in the Pfam database represent evolutionary conserved structure that may be relevant for a protein's function. The hidden Markov models (HMMs) built from the Pfam alignments are useful for establishing if a protein belongs to an existing protein family. This is even the case if homology by alignment would be low. Once, for example, a protein which is involved in a certain character (e.g. sensitivity to drought) is recognized, and, for example, impairment of its expression imparts a enhanced trait (e.g. increased resistance to drought), other proteins in the same protein family can be identified by the skilled person by comparing the amino acid sequence of a protein (and encoded by candidate DNA) against the Hidden Markov Model which characterizes the Pfam domain (in the current invention Pfam HECT PF00632 model) using HMMER software (http://hmmer.janelia.org/'version. HMMER version 3.0 was released on March 28, 2010).

After establishment of the presence of a Pfam HECT domain (PF00632) as described above, a candidate protein also has to meet the requirement of comprising at least on Superfamily ARM repeat (HMM model SSF48371; http://supfam.org/SUPERFAMILY/cgi-bin/scop.cgi?ipid=SSF48371, as can be established by, for example using the InterProScan application (http://www.ebi.ac.uk/Tools/pfa/iprscan/; Quevillon et al. (2005) 33(2) W116-W120; E. M. Zdobnov and R. Apweiler (2001) Bioinformatics, 17, 847-848). Quevillon and colleagues describe that the InterProScan is a tool that combines different protein signature recognition methods from the InterPro consortium member databases into one resource, with distinct publicly available databases in the application. Protein as well as DNA sequences can be analyzed. A web-based version is accessible for academic and commercial organizations from the EBI (http://www.ebi.ac.uk/InterProScan/).

The SUPERFAMILY annotation is based on a collection of hidden Markov models, which represent structural protein domains at the SCOP superfamily level. A superfamily groups together domains which have an evolutionary relationship. The annotation is produced by scanning protein sequences from over 1,400 completely sequenced genomes against the hidden Markov models.

All software is applied under default settings.

In summary, a Hidden Markov model for the HECT domain (PF00632 model http://pfam.sanger.ac.uk/family?acc=PF00632) was obtained from the Pfam database (version 24 from http://pfam.sanger.ac.uk/) and placed into a separate file. The HMMER software was used to determine that the amino proteins sequences are characterized by the Pfam HECT domain. In addition, the filtered protein set was further reduced by employing the SuperFamily package (using the SSF48371 model http://supfam.org/SUPERFAMILY/cgi-bin/scop.cgi?ipid=SSF48371) from the InterProScan application (http://www.ebi.ac.uk/Tools/pfa/iprscan/) to mine for ARM repeats.

(Plant) Proteins meeting both requirements (having a Pfam HECT PF00632 domain and a SuperFamily SSF48371 model Arm repeat), are proteins according to the invention as claimed; and impairment of the expression thereof may be useful in providing improved/increased drought resistance to the plant, and examples of such proteins and cDNA are disclosed herein. The skilled person is well aware on how to determine and test based on the information provided above.

Without being bound by theory, the current inventors speculate that the presence of this combination of domains in the protein according to the invention as claimed increases sensitivity of the plants for drought, and that impairment of the expression of such proteins having these domains, improves resistance of a plant to drought.

Impairment at the transcriptional level can be the result of the introduction of one or more mutations in transcription regulatory sequences, including promoters, enhancers, initiation, termination or intron splicing sequences. These sequences are generally located 5' of, 3' of, or within the coding sequence of the genes according to the claimed invention. Independently, or at the same time, impairment of expression can also be provided by deletion, substitution, rearrangement or insertion of nucleotides in the coding region of the genes.

For example, in the coding region, nucleotides may be substituted, inserted or deleted leading to the introduction of one, two or more premature stop-codons. Also, insertion, deletion, rearrangement or substitution can lead to modifications in the amino acid sequence encoded, and thereby providing for impaired expression of functional UPL3 protein. Even more, large parts of the genes may be removed, for example, at least 10%, 20%, 30%, 40%, 50%, 60%, 70%, 80%, 90% or even 100% of the (coding region) of the gene is removed from the DNA present in the plant, thereby impairing the expression of functional UPL3 protein.

Alternatively, one, two, three of more nucleotides may be introduced in the gene or genes encoding for a UPL3 protein, either leading to for example a frame-shift, or leading to the introduction of a sequence encoding additional amino acids, or the introduction of a sequence not encoding amino acids, or the introduction of large inserts, thereby impairing the provision/expression of functional UPL3 protein.

In other words, deletion, substitution or insertion of nucleotide(s) in a nucleotide sequence encoding a UPL3 protein, as described above, may lead to, for example, a frame shift, an introduction of a stop codon, or the introduction of a non-sense codon. In particular the introduction of a stop codon and the introduction of a frame shift mutation are generally accepted as efficient ways to produce a knockout plant, that is, a plant with reduced, repressed or deleted expression and/or activity of a specific protein.

A frame shift mutation (also called a framing error or a reading frame shift) is a genetic mutation caused by indels (insertions or deletions) of a number of nucleotides that is not evenly divisible by three in a nucleotide sequence. Due to the triplet nature of gene expression by codons, the insertion or deletion can change the reading frame (the grouping of the codons), resulting in a completely different translation from the original. The earlier in the sequence the deletion or insertion occurs, the more altered the protein produced is. A frame shift mutation will in general cause the reading of the codons after the mutation to code for different amino acids, but there may be exceptions resulting from the redundancy in the genetic code. Furthermore, the stop codon ("UAA", "UGA" or "UAG") in the original sequence will not be read, and an alternative stop codon may result at an earlier or later site. The protein produced may be abnormally short or abnormally long.

The introduction of a stop codon in a nucleotide sequence encoding a UPL3 protein as defined herein may result in a premature stop of transcription, which generally results in a truncated, incomplete, and non-functional UPL3 protein. Preferably, the stop codon is introduced early in the transcription direction. The earlier in the nucleotide sequence the stop codon is introduced, the shorter and the more altered the protein produced is. The introduction of a nonsense codon in a nucleotide sequence encoding a UPL3 protein may result in transcript mRNA wherein e.g. one codon no longer codes for the amino acid as naturally occurring in UPL3, for example a codon that normally codes for an amino acid which is essential for a UPL3 protein to be functional. Hence, such UPL3 protein may not be functional.

In other words, the impairment may comprise mutating one or more nucleotides in the genes disclosed herein resulting either in the presence of less or even in the total absence of protein expression product (i.e. the absence of protein that would be obtained when the genes according to the claimed method of the invention were not modified as described above), or in the presence of non-functional protein.

Therefore, in one embodiment of the method disclosed herein, the impairment is the consequence of one or more mutations in said gene resulting in the presence of less protein expression product or absence of a protein expression product.

The term inhibition/presence of less as used herein relates to a reduction in protein expression of at least 10%, 20%, 30%, 40%, 50%, 60%, 70%, 80%, 90% or even 99 % in comparison to a control plant, in which the expression is not impaired. The term "absence of protein expression" refers to the virtual absence of any expression product, for example less than 5%, 4%, 3%, 2% or even less than 1% in comparison to the control.

As will be understood by a skilled person, a mutation may also be introduced in a nucleotide sequence encoding UPL3 as defined herein by the application of mutagenic compounds, such as ethyl methanesulfonate (EMS) or other compounds capable of (randomly) introducing mutations in nucleotide sequences. Said mutagenic compounds or said other compound may be used as a means for creating plants harboring a mutation in a nucleotide sequence encoding a UPL3 protein.

Alternatively, the introduction of a mutation in a nucleotide sequence encoding a (UPL3) protein according to the claimed invention is effected by the introduction of transfer-DNA (T-DNA) in the nucleotide sequence encoding such protein, for instance T-DNA of the tumor-inducing (Ti) plasmid of some species of bacteria such as Agrobacterium tumefaciens. A T-DNA element may be introduced in said nucleotide sequence, leading to either a non-functional protein or to the absence of expression of the protein, consequently decreasing the need for water of a plant obtained by the method according to the invention (see for example Krysan et al. 1999 The Plant Cell, Vol 11. 2283-2290). Likewise advantage can be taken from the use of transposable element insertion (See for Example Kunze et al (1997) Advances in Botanical Research 27 341- 370 or Chandlee (1990) Physiologia Planta 79(1) 105-115).

Preferably, introducing a mutation in a nucleotide sequence encoding a protein according to the claimed invention is performed by targeted nucleotide exchange (TNE), for instance as described in WO2007073170. By applying TNE, specific nucleotides can be altered in a nucleotide sequence encoding UPL3, whereby, for instance, a stop codon may be introduced which may for instance result in a nucleotide sequence encoding a truncated UPL3 protein with decreased or disappeared activity.

In another embodiment there is provided a method as disclosed above wherein the impairment of expression of functional UPL3 protein is caused by expression of non-functional protein. As explained above, a skilled person has no problem in determining functionality of the UPL3 genes For example, he may perform complementation studies, by introducing the control gene, without any modifications, into a plant in which the expression of a UPL3 protein has been impaired and study drought resistance.

Alternatively he may perform experiments analogous to those experiments described below in the examples, and determine drought resistance in a plant in which one or more mutations were introduced in the UPL3 genes by comparison to a suitable control/wild-type plant.

Impairment can also be provided at the translational level, e.g. by introducing a premature stop-codon or by posttranslational modifications influencing, for example, protein folding.

Independent of the mechanism, impairment according to the present invention is indicated by the absence or reduced presence of a functional UPL3 protein. As explained above the term inhibition of expression or reduced presence as used herein relates to a reduction in protein expression of at least 10%, 20%, 30%, 40%, 50%, 60%, 70%, 80%, 90% or even 99% in comparison to a control plant, in which the expression is not impaired. The term "absence of protein expression" refers to the virtual absence of any expression product, for example less than 5%, 4%, 3%, 2% or even less than 1% in comparison to the control.

According to another embodiment, impairment is caused by gene silencing, for example with RNA interference or RNA silencing.

With the help of molecular biology methods readily available to the skilled person, impairment of the genes can also be accomplished by gene silencing, for example using RNA interference techniques, dsRNA or other expression silencing techniques (see for example, Kusaba et. al (2004) Current Opinion in Biotechnology 15:139-143, or Preuss and Pikaard (2003) in RNA Interference (RNAi)~Nuts & Bolts of siRNA Technology (pp.23-36), ©2003 by DNA Press, LLC Edited by: David Engelke, Ph.D.) or, as already discussed above, knocking out.

In another preferred embodiment, and as already discussed above, there is provided for a method according to the invention wherein the impairment is caused by insertion, deletion and/or substitution of at least one nucleotide. For example, 1, 2, 3...10, 40, 50, 100, 200, 300, 1000, or even more nucleotides may be inserted, deleted or substituted in the UPL3 genes. Also anticipated are combinations of insertion, deletion and/or substitution, either in the coding or in the non-coding regions of the gene.

In another embodiment of the method disclosed herein the method comprises the step of impairing the expression in said plant of more than 1, for example 2, 3, 4, 5, or all genes encoding a UPL3 protein.

In this embodiment, the expression of more than one gene as described above, and present in a particular plant is impaired. For example the expression of one, two, three, four, or all of the genes encoding a UPL3 protein present in a plant, is impaired. By impairing the expression of more genes as described above at the same time (when present in a plant) even more improved drought resistance can be achieved.

In another embodiment, the plant provided by the method according to the invention can be used for the production of further plants and or plant products derived there from. The term "plant products" refers to those material that can be obtained from the plants grown, and include fruits, leaves, plant organs, plant fats, plant oils, plant starch, plant protein fractions, either crushed, milled or still intact, mixed with other materials, dried, frozen, and so on. In general such plant products can, for example be recognized by the presence of a gene as disclosed herein so modified that the expression of a functional protein is impaired, as detailed above.

Expression and/or activity of the UPL3 protein according to the invention is impaired (e.g. reduced, repressed or deleted) in a plant belonging to the *Brassicaceae* family including *Brassica napus* (rape seed) or *Solanaceae-family,* including tomato, or *Curcurbitaceae* family, including melon and cucumber, Preferably the method according to the invention is applied in tomato, melon, or cucumber, thereby providing a plant with decreased need for water or improved resistance to drought in comparison to a corresponding non-transformed plant.

Also provided is a plant cell, plant or plant product derived thereof obtainable by the method according to the claimed invention, and wherein said plant cell, plant or plant product shows reduced expression of functional UPL3 protein, compared to a control plant not subjected to the method according to the claimed invention.

Also provided is a plant cell, plant or plant product derived thereof, characterized in that in said plant cell, plant or plant product derived thereof the expression of at least one, preferably all genes encoding UPL3 protein, such as when the cDNA sequence corresponding to the mRNA sequence transcribed from said at least one gene comprises the sequence shown in SEQ ID NO:1, or the cDNA corresponding to mRNA sequence transcribed from said gene comprises the sequences with at least 40%, 50%, 60%, 70%, 80%, 90%, 95 % identity with the sequence of SEQ ID NO: 1, preferably over its entire length, and/or wherein the amino acid sequence encoded by said at least one gene comprises the sequence shown in SEQ ID NO:2, and amino acid sequence sequences with more than 30%, 35%, 40%, 50%, 60%, 70%, 80%, 90%, 95 % identity with the sequence of SEQ ID NO:2 and/or wherein the amino acid sequence encoded by said at least one gene comprises at least one Pfam HECT domain (PF00632) and at least one Superfamily ARM repeat (model SSF48371) as defined above, is impaired. Preferably the plant is not the *Arabidopsis* T-DNA insertion mutant as described in the examples.

In another aspect the invention is directed to a use of a gene or nucleotide sequence wherein the cDNA corresponding to the mRNA sequence transcribed from said gene comprises the sequence shown in SEQ ID NO:1, or the cDNA corresponding to mRNA sequence transcribed from said gene comprises the sequences with at least 80%, 90%, 95 % identity therewith and/or wherein the amino acid sequence encoded by said gene comprises the sequence shown in SEQ ID NO:2, and amino acid sequence sequences with more than 30%, 35%, 40%, 50%, 60%, 70%, 80%, 90%, 95 % identity therewith and/or wherein the amino acid sequence encoded by said gene comprises at least one Pfam HECT domain (PF00632) and at least one Superfamily ARM repeat (model SSF48371) as defined above, for providing increased drought resistance to a plant.

The gene described can be used as a target for improving drought resistance in a plant, in accordance with the disclosure herein.

Provided herein is a use of a UPL3 sequence having SEQ ID No.1 or 2 of the Arabidopsis thaliana species in the screening for drought resistance in Arabidopsis thaliana plants. In addition, a use is provided wherein the UPL3 sequence is an analogous sequence to SEQ ID No.1 or 2 of an other plant species and wherein the screening is in plants of the other plant species. Furthermore, a method is provided for screening plants or plant cells with improved drought resistance comprising the steps of:
- providing a heterogenic population of plant cells or plants of the Arabidopsis thaliana species;
- providing a UPL3 sequence having SEQ ID No.1 or 2;
- determining the sequence of at least part of the UPL3 gene of the plants cells or plants;
- comparing the determined UPL3 sequences from the plant cells or plants with the provided UPL3 sequence;
- identifying plant cells or plants wherein the UPL3 sequence comprises a mutation. Alternatively, in the method, the plant cells or plants that are provided are of an other species, and wherein the UPL3 gene sequence that is provided is an analogous sequence of the other species.

Hence, by using the UPL3 sequence of SEQ ID No.1 or SEQ ID No.2 of the species *Arabidopsis thaliana,* or an analogous sequence thereof from an other species, mutated UPL3 sequences can be identified in the plant species that may provide improved drought resistance. An analogous sequence, in an other species, of the UPL3 sequence SEQ ID No.1 or SEQ ID No.2 of the species *Arabidopsis thaliana* is defined as a sequence having at least 40%, 50%, 60%, 70%, 75%, 80%, 85%, 90%, 95%, or at least 99%, sequence identity therewith. The analogous UPL3 protein may have substantially the same function as SEQ ID No.1 or SEQ ID No.2.

In the method, a heterogenic population of plant cells or plants of the species is provided. The heterogenic population may for example be provided by subjecting plant cells to a mutagen that introduces random mutations thereby providing a heterogenic population of plant cell. Hence, the heterogenic population may be derived from a single plant variety, which is subjected to random mutagenesis in order to obtain a variety of mutations in the offspring thereby providing a heterogenic population. Many mutagens are known in the art, e.g. ionic radiation, UV-radiation, and mutagenic chemicals such as azides, ethidium bromide, or ethyl methanesulfonate (EMS). Hence the skilled person knows how to provide for a heterogenic population of plants or plant cells. Also, the skilled person may also provide a variety of plants as a heterogenic population, i.e. not a single variety from a species. A variety of plants show genetic variety, they are not genetically identical, but because the plants are from the same species they are substantially identical. In any case, a heterogenic population of plant cells or plants may have at least 95%, 96%, 97%, 98%, 98%, 99%, 99.5% or at least 99,9% sequence identity.

By determining at least part of the sequence of the UPL3 gene sequence with the sequence of the plants or plant cells from the heterogenic population, and subsequently comparing these sequences with the provided UPL3 gene sequence (the reference), plant cells or plants can be identified that comprise a mutation in the UPL3 gene sequence. It is understood that such a comparison can be done by alignment of the sequences and that a mutation is a difference in respect of at least one nucleic acid or amino acid position in the analogous (reference) UPL3 sequence of the plant species. In this way, plants or plant cells are identified that have mutations in the UPL3 gene (e.g. insertions, deletions, substitutions) that may provide improved drought resistance.

Preferably, plants are selected that have mutations that would result in an impairment of expression of a functional UPL3 protein, such as already outlined above. Mutations that would impair expression of a functional UPL3 protein may be mutations that would disrupt the open reading frame (introduce a frame shift or a stop codon), or disrupt or otherwise alter the function of the encoded protein by altering nucleotides in codons encoding amino acids that are essential for the proper functioning of the protein, thereby leading to modified (e.g. increased) resistance to draught in comparison to the non-altered protein. The method may also be used for example in the screening and selection of plants that have been subjected to genetic modification which targets the UPL3 gene sequence as outlined above. Also, the UPL3 sequence may also be used in a screening assay, in which a (heterogenic) population of plants are subjected to drought. Plants that show improved drought resistance may provide

In another embodiment, the use is provided of at least part of UPL3 having SEQ ID No.1 or SEQ ID No.2 of the *Arabidopsis thaliana* species as a marker for breeding drought resistant Arabidopsis thaliana plants. Also, the UPL3 sequence may be of an analogous sequence of an other species wherein the marker is for breeding drought resistant plants of the other plant species.

Described herein is the use of a plant, plant cell or plant product wherein expression of functional UPL3 protein is impaired, wherein the functional UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed for growing under drought stress conditions, wherein said drought stress conditions cause a control plant, plant cell or plant product wherein expression of said functional UPL3 protein is not impaired to show signs of drought stress such as wilting signs earlier than the plant, plant cell, or plant product wherein expression of functional UPL3 protein is impaired.

Described herein is a plant, plant cell or plant product obtainable or obtained by the method taught herein. Additionally, described herein is a seed derived from such plant.

Described herein is a plant, plant cell, or plant product wherein expression of functional UPL3 protein is impaired, wherein the functional UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed. Said plant, plant cell or plant product may, for example, comprise a disrupted endogenous UPL3 gene.

The plant, plant cell or plant product may be any plant or plant cell, or may be derived from any plant, such as monocotyledonous plants or dicotyledonous plants, but most preferably the plant belongs to the family *Solanaceae.* For example, the plant may belong to the genus *Solanum* (including *lycopersicum*)*, Nicotiana, Capsicum, Petunia* and other genera. The following host species may suitably be used: Tobacco (*Nicotiana* species, e.g. *N. benthamiana, N. plumbaginifolia, N. tabacum,* etc.), vegetable species, such as tomato (*Solanum lycopersicum*) such as e.g. cherry tomato, var. *cerasiforme* or currant tomato, var. *pimpinellifolium)* or tree tomato (*S. betaceum,* syn. *Cyphomandra betaceae*)*,* potato (*Solanum tuberosum*)*,* eggplant (*Solanum melongena*)*,* pepino (*Solanum muricatum*), cocona (*Solanum sessiliflorum)* and naranjilla (*Solanum quitoense),* peppers (*Capsicum annuum, Capsicum frutescens*, *Capsicum baccatum*)*,* ornamental species (e.g. *Petunia hybrida, Petunia axillaries, P. integrifolia*).

Alternatively, the plant may belong to any other family, such as to the *Cucurbitaceae* or *Gramineae.* Suitable host plants include for example maize/corn (Zea species), wheat (*Triticum* species), barley (e.g. *Hordeum vulgare*)*,* oat (e.g. *Avena sativa*)*,* sorghum (*Sorghum bicolor), rye* (*Secale cereale),* soybean (*Glycine* spp, e.g. *G*. *max*)*,* cotton (*Gossypium* species, e.g. *G. hirsutum, G. barbadense), Brassica* spp. (e.g. *B. napus, B. juncea, B. oleracea, B. rapa,* etc), sunflower (*Helianthus annus*)*,* safflower, yam, cassava, alfalfa (*Medicago sativa*)*,* rice (*Oryza* species, e.g. O. *sativa indica* cultivar-group or *japonica* cultivar-group), forage grasses, pearl millet (*Pennisetum* spp. e.g. *P. glaucum*)*,* tree species (*Pinus,* poplar, fir, plantain, etc), tea, coffea, oil palm, coconut, vegetable species, such as pea, zucchini, beans (e.g. *Phaseolus* species), cucumber, artichoke, asparagus, broccoli, garlic, leek, lettuce, onion, radish, turnip, Brussels sprouts, carrot, cauliflower, chicory, celery, spinach, endive, fennel, beet, fleshy fruit bearing plants (grapes, peaches, plums, strawberry, mango, apple, plum, cherry, apricot, banana, blackberry, blueberry, citrus, kiwi, figs, lemon, lime, nectarines, raspberry, watermelon, orange, grapefruit, etc.), ornamental species (e.g. Rose, Petunia, Chrysanthemum, Lily, *Gerbera* species), herbs (mint, parsley, basil, thyme, etc.), woody trees (e.g. species of *Populus, Salix, Quercus, Eucalyptus*)*,* fibre species e.g. flax (*Linum usitatissimum*) and hemp (*Cannabis sativa*)*,* or model organisms, such as *Arabidopsis thaliana.*

Preferred hosts are "crop plants", i.e. plant species which is cultivated and bred by humans. A crop plant may be cultivated for food purposes (e.g. field crops), or for ornamental purposes (e.g. production of flowers for cutting, grasses for lawns, etc.). A crop plant as defined herein also includes plants from which non-food products are harvested, such as oil for fuel, plastic polymers, pharmaceutical products, cork and the like.

Preferably, the plant, plant cell or plant product described herein is not an *Arabidopsis thaliana* or *Brachypodium* plant, plant cell or plant product.

The plant, plant cell or plant product described herein may, for example, be a *Solanum lycopersicum* or *Brassica rapa* plant, plant cell or plant product.

Thus, the disclosure pertains, for example, to a *Solanum lycopersicum, Gossypium hirsutum, Glycine max, Triticum* spp., *Hordeum vulgare., Avena sativa, Sorghum bicolor, Secale cereale,* or *Brassica napus* plant, plant cell, or plant product wherein expression of functional UPL3 protein is impaired, wherein the functional UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said functional UPL3 protein is not expressed. Said plant, plant cell, or plant product may comprise a disrupted endogenous UPL3 gene.

### Examples

### Example 1 Drought test

Arabidopsis thaliana (At) seeds transformed with Agrobacterium tumefaciens vector pROK2, leading to the absence of functional UPL3 protein (NASC ID: N670558 , AGI code At4g38600 and SALK_037636C; hereafter referred to a mutant seeds or mutant plants) were obtained from the Nottingham Arabidopsis Stock Centre (NASC; School of Biosciences, University of Nottingham, Sutton Bonington Campus, Loughborough, LE12 5RD United Kingdom). As control At Col-0 (Columbia, N60000); hereafter referred to as control seed or plant) were used.

### Growth medium:

A soil mixture comprising one part of sand and vermiculite and two parts of compost was used (sand:vermiculite:compost = 1:1:2). This mixture increases the water percolation hence facilitates uniform water uptake by each pot and better water drainage. Before sowing, the seeds were kept at 4°C for 3 days under dark and humid conditions for stratification.

Both mutant and control seeds were sown in a rectangular tray containing 8 x 5 = 40 pots of ~4cm diameter with density of 5 plants per pot. Nutrient solution (EC=1.5) was supplied to all the plants from the bottom of the pots in the tray 10 days after germination (DAG), and at 15 DAG the plants were subjected to drought (for 15, 16, 17 or 18 days) by transferring the pots to dry trays. Subsequently, plants were rehydrated and observed for recovery after 1 week.

Three pot replicates of each genotype were included in the pre-drought screening.

Total time needed for a complete test was approx. 36-39 days.

### Drought assay examination

Once the plants reached the 2 true leaves stage they were thinned to maintain exactly 5 plants per pot. At 10 DAG, plants received nutrition (EC=1.5) and at 15 DAG each pot was moved to a dry tray. From this day onwards the plants did not receive any water. Every day the plants, especially the control (or wild type) (Col-0) were observed for wilting signs. On the 15^{th} day of drought (DOD), Col-0 wilted completely and did not recover upon rehydration. We determined this day as its permanent wilting point (PWP). From this day onwards one replicate from the mutant was rehydrated and observed for recovery signs and pictures were taken. The mutant showed survival for at least 2 days more under drought compared to the control and was subjected for further rigorous screening.

### Example 2 Drought test

### Growth medium:

The same mutant and control plants as in Example 1 were grown in similar tray set-up as described above in the pre-screening test. Plants were stressed by withholding water from 15 DAG until the control reached its PWP. During this period every alternate day pots were shuffled within the trays to reduce the position effects and allow uniform evaporation. On day 15 DOD, control plants reached PWP and did not recover upon rehydration. One pot replicate from the mutant was rehydrated everyday from 15 DOD onwards and checked for drought stress recovery. Pictures were taken and recovery was scored. The mutant showed recovery from drought stress for at least 3 days more after the control reached its PWP.

Figure 1 shows a photograph comparing mutant and control (left), demonstrating the superior effect of the mutant (right column) with respect to resistance to drought stress.

### Example 3: Drought test

**Plant material.** TDNA insertion lines with a disrupted AT4G38600 gene (SALK_037636C) were obtained from the Nottingham Arabidopsis Stock Centre (NASC). Complementation lines were produced by stable transformation of *Arabidopsis thaliana* plants using floral dip transformation (Bent et al., 2006. Methods Mol. Biol. Vol. 343:87-103). Homologs of the *Arabidopsis thaliana* (AT4G38600) UPL3 gene were identified from several crop species, including *Solanum lycopersicum* (tomato) and *Oryza sativa* (rice) and the model species *Arabidopsis thaliana* (UPL4; AT5G02880).

**Table 1. Homologs of the Arabidopsis thaliana UPL3 gene**

| ***Annotation*** | ***Arabidopsis thaliana*** | ***Solanum lycopersicum*** | ***Oryza sativa*** |
|---|---|---|---|
| Ubiquitin protein ligase 3 (UPL3) | At4g38600 (SEQ ID NO:1 & 2) | Slg81026 (SEQ ID NO:5 & 6) | Os05g03100 (SEQ ID NO:9 & 10) |
| | At5g02880 (SEQ ID NO:3 & 4; UPL4) | Slg98247 (SEQ ID NO:7 & 8) | |

**Table 2. Percentage of nucleic acid sequence identity between the Arabidopsis thaliana UPL3 cDNA sequence (SEQ ID NO:1) and cDNA sequences of homologues in Arabidopsis thaliana (At5g02880 (UPL4); SEQ ID NO:3), Solanum lycopersicum (SIg98247; SEQ ID NO:7 and SIg81026; SEQ ID NO:5), and Oryza sativa (Os05g03100; SEQ ID NO:9)(first column); and percentage of amino acid sequence identity between the Arabidopsis thaliana UPL3 protein sequence (SEQ ID NO:2) and protein sequences of homologues in Arabidopsis thaliana (At5g02880 (UPL4); SEQ ID NO:4), Solanum lycopersicum (SIg98247; SEQ ID NO:8 and SIg81026; SEQ ID NO:6), and Oryza sativa (Os05g03100; SEQ ID NO:10)(second column).**

| | **Nucleotide sequence** | **Amino acid sequence** |
|---|---|---|
| **At5g02880** | 62 | 40 |
| **SIg98247** | 71 | 39 |
| **SIg81026** | 61 | 39 |
| **Os05g03100** | 66 | 33 |

**Drought assay.** Wild-type, TDNA knock-out and complementation lines were sown in a replicated blocked design in 50-cell seedlings trays containing a 2:1:1 mix of Metro-Mix 852 soilless medium, fine sand and vermiculite. Planted trays were placed at 4°C for three days to break dormancy and then transferred to a growth chamber (16 h 22/20°C, 50% rH) for germination and establishment. Complementation lines were sprayed with a glufosinate formulation (20 mg glufosinate, 20 µL Silwet surfactant, 200 mL water) once they had fully expanded cotyledons to assure that only transformed lines were selected. Following this treatment, seedlings in each cell were thinned to a single plant. Once plants reached the 4-6 true leaf stage they were acclimated to greater vapor pressure deficit conditions to promote even drought stress (28/26°C, 25% rH) and unusually small plants were identified for removal prior to drought treatment. Planting trays were soaked with water and then allowed to drain, leaving all cells at pot capacity. Entire trays were watered once half of the wild-type plants in any given tray appeared to be at their permanent wilting point (1.5 - 2 weeks of drought treatment). Plants were allowed to recover over a few days and survival was recorded, with pre-identified abnormally small plants omitted from further analyses.

**Statistical analysis.** Statistical significance of differing probabilities of survival over this drought treatment was assessed by applying the test of equal or given proportions in the statistical software program, R (http://www.r-project.org/). The function prop.test was used to test the null hypothesis that the proportions of surviving plants between mutant and wild-type (one-tailed), or alternatively, between insertion mutant lines containing or not containing complementing transgenes (two-tailed), were equal.

### Results

Figure 2 shows the drought resistant phenotype of the UPL3 knockout (*Arabidopsis* At4g38600 insertion mutant) as compared to the drought sensitive phenotype of a control (wild-type) plant.

The *Arabidopsis* At4g38600 insertion mutant survived drought significantly better (p < 0.05) than wild-type (Col-0) plants or At4g38600 insertion mutants complemented with the coding sequence (CDS) of At4g38600 (SEQ ID NO:1; positive control), and homologs from *Arabidopsis thaliana* (At5g02880; SEQ ID NO:3), *Solanum lycopersicum* (SEQ ID NO:5 and SEQ ID NO:7) or *Oryza sativa* (SEQ ID NO:9). Figure 3 demonstrates that an insertion mutation in the UPL3 gene produces a drought resistant phenotype. Moreover, it also indicates that homologs of this gene from monocot and dicot species operate to restore the normal drought-susceptible phenotype. Hence, these homologs are assumed to perform the same function in drought tolerance in their respective crop species. The observation that both monocot and dicot UPL3 genes can restore drought susceptibility when inserted into the UPL3 mutant of *Arabidopsis* suggests that a reduced activity of the protein encoded by the UPL3 gene renders drought tolerant phenotypes throughout the entire plant kingdom. Hence, prediction of UPL3 (based on homology searches and characteristic domain [HECT] and Armadillo repeat sequences) will allow identification of plant UPL3 homologs in plant species. Subsequently, one can use well-known methods to reduce protein activity of these plant homologs (e.g. mutagenesis, TDNA or transposon insertion, RNAi, etc) to obtain drought resistant plants. Grey bars have significantly lower values (p < 0.05) than black bars.

### Example 4 Drought resistance in tomato

**Plant material.** A novel mutation in the tomato gene Solyc10g055450 (Slg98247; SEQ ID NO:7) was generated using EMS and identified through EMS screening. The mutation consisted of an amino acid change of valine (hydrophobic properties) to glutamic acid (negatively charged amino acid) (in position 158 of the protein). A segregating M2 population containing homozygous, heterozygous and wild-type allele were used for all drought experiments.

A second mutation was identified in the same tomato gene, causing an amino acid change of aspartic acid (negatively charged amino acid) to glutamic acid (negative charged amino acid) (in position 114 of the protein). Due to the similarity in biochemical properties, this mutation was unlikely to cause significant changes to the protein properties and was therefore used as a negative control in the drought assays. Sift (Ng and Henikoff, 2003 - Nucl. Acids Res. 31: 3812-3814) analysis showed that this mutation is likely to be tolerated. A segregating M2 population containing homozygous, heterozygous and wild-type allele were used for all drought experiments.

**Drought assay.** Tomato seedlings that were homozygous, heterozygous or wild-type for the described V158E mutation were grown in 2.5 inch plastic pots containing a 2:1:1 mix of Metro-Mix 852 soilless medium, fine sand and vermiculite in a growth chamber (16 h 22/20°C, 50% rH. Upon establishment, seedlings were acclimated to greater vapor pressure deficit conditions to promote even drought stress (28/26°C, 25% rH). Pots were soaked with water and then allowed to drain, leaving all plants at pot capacity. Plants were subjected to a drought stress period of 1 week and then watered and allowed to recover for 24 h, when survival was assessed.

**Statistical analysis.** Statistical significance of differing probabilities of survival over this drought treatment was assessed by apply the test of equal or given proportions in the statistical software program, R (http://www.r-project.org/). The function prop.test was used to test the null hypothesis that the proportions of surviving plants between homozygous and wild-type mutants (one-tailed) were equal.

### Results

Tomato plants, homozygous for the V158E mutation in SIg98247 survived the drought treatment significantly better (p < 0.1) compared to the wild-type allele, indicating that this alteration of the protein leads to a drought tolerant phenotype in tomato (Fig. 4). As expected the additional mutation in SIg98247 (D114E) did not show any drought related phenotype (all plants from the segregating M2 population were equally drought susceptible).

## Claims

1. Method for improving drought resistance of a plant compared to a control plant, comprising the step of impairing expression of an endogenous UPL3 protein in a plant, plant protoplast or plant cell, wherein said endogenous UPL3 protein is encoded by a nucleic acid comprising a nucleic acid sequence having at least 80% identity with SEQ ID NO:1, or wherein the UPL3 protein has the amino acid sequence of at least one of SEQ ID NO: 2, 6 and 8,
wherein the plant, plant protoplast or plant cell belongs to at least one of the *Brassicaceae* family and the *Solanaceae* family, and wherein the UPL3 expression is impaired by at least one of:
i) a step of mutating a nucleic acid sequence encoding said endogenous UPL3 protein; and
ii) a step of gene silencing,
and wherein the method further comprises the steps of
regenerating said plant; and
determining improved drought resistance of said plant as compared to the control plant.

2. Method according to claim 1, wherein the plant, plant protoplast or plant cell is at least one of an *Arabidopsis thaliana* and a *Solanum lycopersicum* plant, plant protoplast or plant cell.

3. Method according to claim 1 or 2, wherein said endogenous UPL3 protein comprises an amino acid sequence comprises
i) at least one Pfam HECT domain according to PF00632; and
ii) four Armadillo repeat sequences.

4. Method according to any one of claims 1 - 3, wherein the endogenous UPL3 protein is a protein that when expressed in an *Arabidopsis thaliana* T-DNA insertion line having a disrupted endogenous UPL3 gene results in a plant with an impaired drought resistance compared to the drought resistance of said *Arabidopsis thaliana* T-DNA insertion line having a disrupted endogenous UPL3 gene in which said endogenous UPL3 protein is not expressed.

5. Method according to any one of claims 1 - 4, wherein mutating said nucleic acid sequence involves an insertion, a deletion and/or substitution of at least one nucleotide.

6. Method according to any one of claims 1-5, comprising the step of impairing expression of two or more endogenous UPL3 proteins in said plant.

7. Method according to any one of claims 1-6, further comprising the step of producing a plant product from the plant having improved drought resistance, and wherein the plant product is selected from the group consisting of leaves, plant organs, plant fats, plant oils, plant starch, plant protein fractions.

## Patentansprüche

1. Verfahren zur Trockenresistenzverbesserung einer Pflanze, verglichen mit einer Kontrollpflanze, umfassend
den Schritt der Beeinträchtigung von Expression eines endogenen UPL3-Proteins in einer Pflanze, einem Pflanzenprotoplasten oder einer Pflanzenzelle, wobei besagtes endogenes UPL3-Protein durch eine Aminosäure kodiert wird, die eine Aminosäuresequenz umfasst, die mindestens 80 % Identität mit der Aminosäuresequenz von SEQ ID. Nr. 1 aufweist, oder wobei das UPL3-Protein die Aminosäuresequenz von mindestens einer der SEQ ID. Nr. 2, 6 und 8 aufweist,
wobei die Pflanze, der Pflanzenprotoplast oder die Pflanzenzelle zu mindestens einer der *Brassicaceae-*Familie und der *Solanaceae*-Familie gehört, und wobei die UPL3-Expression durch mindestens eines der folgenden beeinträchtigt wird:
i) einem Schritt der Mutation einer Nukleinsäuresequenz, die besagtes endogenes UPL3-Protein kodiert; und
ii) einem Schritt der Gensilenzierung,
und wobei das Verfahren ferner die Schritte umfasst von
Regenerieren besagter Pflanze; und
Bestimmen verbesserter Trockenresistenz besagter Pflanze, als verglichen mit der Kontrollpflanze.

2. Verfahren nach Anspruch 1, wobei die Pflanze, der Pflanzenprotoplast oder die Pflanzenzelle mindestens eine/r ist von einer/m *Arabidopsis thaliana-* und einer *Solanum lycopersicum*-Pflanze, -Pflanzenprotoplast oder -Pflanzenzelle.

3. Verfahren nach Anspruch 1 oder 2, wobei das endogene UPL3-Protein eine Aminosäuresequenz umfasst, umfassend
i) mindestens eine Pfam-HECT-Domäne gemäß PF00632; und
ii) vier Armadillo-Wiederholungssequenzen.

4. Verfahren nach einem der Ansprüche 1-3, wobei das endogene UPL3-Protein ein Protein ist, das, wenn es in einer *Arabidopsis thaliana*-T-DNA-Insertionslinie, die ein gestörtes endogenes ULP3-Gen aufweist, exprimiert wird, in einer Pflanze mit einer beeinträchtigten Trockenresistenz resultiert, verglichen mit der Trockenresistenz der genannten *Arabidopsis thaliana-*T -DNA-Insertionslinie, die ein gestörtes endogenes UPL3-Gen aufweist, in der besagtes endogenes UPL3-Protein nicht exprimiert wird.

5. Verfahren nach einem der Ansprüche 1-4, wobei Mutation besagter Nukleinsäuresequenz eine Insertion, eine Deletion und/oder Substitution mindestens eines Nukleotids einbezieht.

6. Verfahren nach einem der Ansprüche 1-5, umfassend den Schritt der Beeinträchtigung von Expression von zwei oder mehr endogenen UPL3-Proteinen in besagter Pflanze umfasst.

7. Verfahren nach einem der Ansprüche 1-6, das ferner den Schritt der Herstellung eines Pflanzenprodukts aus der Pflanze, die verbesserte Trockenresistenz aufweist, umfasst, wobei das Pflanzenprodukt ausgewählt ist aus der Gruppe bestehend aus Blättern, Pflanzenorganen, Pflanzenfetten, Pflanzenölen, Pflanzenstärke und Pflanzenproteinfraktionen.

## Revendications

1. Procédé pour améliorer la résistance à la sécheresse d'une plante par rapport à une plante témoin, comprenant l'étape consistant à inhiber l'expression d'une protéine UPL3 endogène dans une plante, un protoplaste végétal ou une cellule végétale, où ladite protéine UPL3 endogène est codée par un acide nucléique comprenant une séquence d'acides nucléiques ayant au moins 80 % d'identité avec la SEQ ID. n° 1, ou où la protéine UPL3 a la séquence d'acides aminés d'au moins l'une des séquences SEQ ID. n° 2, 6 et 8,
où la plante, le protoplaste végétal ou la cellule végétale appartient à au moins l'une parmi la famille des *Brassicaceae* et la famille des *Solanaceae,* et où l'expression de la UPL3 est inhibée par au moins l'une des étapes suivantes :
i) une étape consistant à muter une séquence d'acides nucléiques codant pour ladite protéine UPL3 endogène ; et
ii) une étape de silençage génique,
et où le procédé comprend en outre les étapes consistant à :
régénérer ladite plante ; et
déterminer l'amélioration de la résistance à la sécheresse de ladite plante par rapport à la plante témoin.

2. Procédé selon la revendication 1, où la plante, le protoplaste végétal ou la cellule végétale est au moins l'une parmi une plante, un protoplaste végétal ou une cellule végétale d*'Arabidopsis thaliana* ou de *Solanum lycopersicum.*

3. Procédé selon la revendication 1 ou 2, où la protéine UPL3 endogène comprend une séquence d'acides aminés comprenant
i) au moins un domaine Pfam HECT selon PF00632 ; et
ii) quatre séquences de répétition Armadillo.

4. Procédé selon l'une quelconque des revendications 1 à 3, où la protéine UPL3 endogène est une protéine qui, lorsqu'elle est exprimée dans une lignée d'insertion d'ADN-T d'*Arabidopsis thaliana* ayant un gène UPL3 endogène inactivé, donne lieu à une plante dont la résistance à la sécheresse est inhibée par rapport à la résistance à la sécheresse de ladite lignée d'insertion d'ADN-T d'*Arabidopsis thaliana* ayant un gène UPL3 endogène inactivé où ladite protéine UPL3 endogène n'est pas exprimée.

5. Procédé selon l'une quelconque des revendications 1 à 4, où la mutation de ladite séquence d'acides nucléiques implique une insertion, une délétion et/ou une substitution d'au moins un nucléotide.

6. Procédé selon l'une quelconque des revendications 1 à 5, comprenant l'étape consistant à inhiber l'expression de deux ou plus protéines UPL3 endogènes dans ladite plante.

7. Procédé selon l'une quelconque des revendications 1 à 6, comprenant en outre l'étape consistant à produire un produit végétal à partir de la plante ayant une résistance améliorée à la sécheresse, et où le produit végétal est choisi dans le groupe constitué par les feuilles, les organes végétaux, les graisses végétales, les huiles végétales, l'amidon végétal, les fractions de protéines végétales.
